# EUROPEAN PATENT SPECIFICATION

(11) **EP 4 532 650 B1**
(45) Date of publication and mention of the grant of the patent: **04.02.2026**
(21) Application number: 23725981.7
(22) Date of filing: 09.05.2023
(51) Int. Cl.: C11D 1/29, C11D 1/34, C11D 1/37, C11D 3/386, C12N 9/96

(54) **COMPOSITION CONTAINING ENZYME**
ENZYME ENTHALTENDE ZUSAMMENSETZUNG
COMPOSITION CONTENTANT UNE ENZYME

(30) Priority: 27.05.2022 WO PCT/CN2022/095572; 30.06.2022 EP 22182330
(43) Date of publication of application: 09.04.2025
(73) Proprietor: Unilever IP Holdings B.V., 6708 WH Wageningen (NL); Unilever Global IP Limited, Wirral, Merseyside CH62 4ZD (GB)
(72) Inventor: GAO, Wei, 6708 WH Wageningen (NL); SHEN, Jun, 6708 WH Wageningen (NL); YIN, Qin, 6708 WH Wageningen (NL)
(74) Representative: Newbould, Frazer Anthony
(86) International application number: PCT/EP2023/062161
(87) International publication number: WO 2023/227356

(56) References cited:
- EP-A1- 0 698 659
- WO-A1-2017/140392
- DE-A1- 3 642 218

## Description

### Technical Field of the Invention

The present invention relates to a composition, particularly a detergent composition comprising an anionically modified alkyl and/or alkenyl phenol polyoxyalkylene ether and an enzyme.

### Background of the Invention

Enzymes are widely used in detergent industry due to their function as highly effective low-temperature catalysts in the stain removal process. Commonly used enzymes are amylases (carbohydrate-containing stains), lipases (fat and edible oil stains), proteases (protein stains) and cellulases (removal of fuzz and pills from cotton fabrics). However, the stability of such enzymes in detergent compositions remains a major obstacle, particularly in liquid compositions. They are prone to decrease in enzyme activity over the lifetime of the detergent composition, leading to reduced stain removal efficiency. Typically, the loss of activity originates from a number of undesirable interactions between the enzymes and the other components of the detergent composition.

Anionic surfactants are commonly used in detergent compositions because of their excellent cleaning capability to help remove oil and greasy substances. However, it is challenging to formulate anionic surfactants together with enzymes since they interact strongly with enzymes by altering their tertiary structure, which results in protein unfolding and loss of activity.

Nowadays, bio-based compounds have gained significant interest due to an increasing demand of sustainable alternatives to petroleum based raw materials. Some consumers prefer compounds with a good environmental profile. For the purposes of environmental sustainability, greener choices of surfactants may be used, especially those derived from raw materials with plant origin.

The present invention has been devised in the light of the above considerations. It has been found unexpectedly that a composition comprising an anionically modified alkyl and/or alkenyl phenol polyoxyalkylene ether and an enzyme provides improved enzyme stability and activity during storage conditions.

### Summary of the Invention

In a first aspect, the present invention is directed to a composition comprising:
a) an anionically modified alkyl and/or alkenyl phenol polyoxyalkylene ether represented by the formula (1) 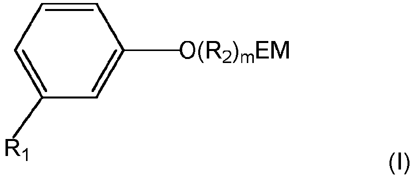 Wherein R₁ is a linear or branched, alkyl or alkenyl group having 11 to 21 carbon atoms; each R₂ is an oxyalkylene group having 2 to 4 carbon atoms; m is an integer from 1 to 50; E is a group comprising one or more of sulfate, phosphate, carboxylate, sulfonate, sulfosuccinate, sulfoacetate, sarcosinate and phosphonate; M is a solubilizing cation selected from sodium, potassium, ammonium, mono-, di-, tri-alkanolamine and mixtures thereof; and
b) an enzyme.

In a second aspect, the present invention is directed to a method for forming a liquid detergent composition or a wash liquor by dispersing a dose of the composition according to any embodiment of the first aspect.

All other aspects of the present invention will more readily become apparent upon considering the detailed description and examples which follow.

### Detailed Description

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Unless specified otherwise, amounts as used herein are expressed in percentage by weight based on the total weight of the composition and is abbreviated as "wt%" or "weight %".

Amounts of wt. % enzymes in the composition refer to wt. % of active protein levels, unless otherwise indicated.

The composition may find use in a variety of cleaning applications. Preferably the composition is a detergent composition. The composition of the present invention may be in any suitable form, for example, a solid such as a powder, a granulated particle and a shaped solid or a liquid. Preferably the composition is a liquid detergent composition. The term "liquid" in the context of this invention denotes that a continuous phase or predominant part of the composition is liquid and that the composition is flowable at 15°C and above. Accordingly, the term "liquid" may encompass emulsions, suspensions, and compositions having flowable yet stiffer consistency, known as gels or pastes. The viscosity of the composition may suitably range from about 200 to about 10,000 mPa·s at 25°C at a shear rate of 21 sec⁻¹. This shear rate is the shear rate that is usually exerted on the liquid when poured from a bottle. Pourable liquid detergent compositions generally have a viscosity of from 200 to 1,500 mPa·s, measured at 25°C at a shear rate of 21 s⁻¹ by a HAAKE Viscometer.

In some embodiments the composition is a laundry detergent composition. The term "laundry detergent" in the context of this invention denotes formulated compositions intended for and capable of wetting and cleaning domestic laundry such as clothing, linens and other household textiles. Examples of liquid laundry detergents include heavy-duty liquid laundry detergents for use in the wash cycle of automatic washing machines, as well as liquid fine wash and liquid colour care detergents such as those suitable for washing delicate garments (e.g. those made of silk or wool) either by hand or in the wash cycle of automatic washing machines. In some embodiments the composition is handwash detergents which involve the consumer using their hands to wash substrates. Fields of use principally involve laundry use (i.e. the hand washing of clothes) and hand dishwash (i.e. the hand washing of dishes and the like). Handwash detergents involve intimate contact of the detergent liquor with the hands during the washing process, whether in laundry or hand dishwash. Laundry detergent composition is particularly preferred.

The composition may be concentrated or dilute. A "concentrated" composition refers to a composition comprising up to 50% by weight of water, for example up to 40%, up to 30% or up to 20%, based on total weight of the composition. Preferably the composition of the present invention is a "dilute" composition. A "dilute" composition refers to a composition comprising greater than 50% by weight of water, for example greater than 60%, greater than 70% or greater than 80%.

### Anionically modified alkyl and/or alkenyl phenol polyoxyalkylene ether

The anionically modified alkyl and/or alkenyl phenol polyoxyalkylene ether is represented by the formula (I): 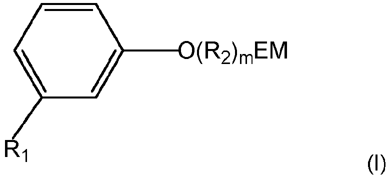 wherein R₁ is a linear or branched, alkyl or alkenyl group having 11 to 21 carbon atoms; each R₂ is an oxyalkylene group having 2 to 4 carbon atoms; m is an integer from 1 to 50; E is a group comprising one or more of sulfate, phosphate, carboxylate, sulfonate, sulfosuccinate, sulfoacetate, sarcosinate and phosphonate; M is a solubilizing cation selected from sodium, potassium, ammonium, mono-, di-, tri-alkanolamine and mixtures thereof.

Preferably R₁ is a linear or branched, alkyl or alkenyl group having 13 to 17 carbon atoms, more preferably R₁ is a linear alkyl or alkenyl group having 13 to 17 carbon atoms. It is particularly preferred that R₁ is a linear C15 alkyl or alkenyl group, more preferably a linear C15 alkyl or alkenyl group comprising 0 to 3 carbon-carbon double bonds.

Preferably, each R₂ is an ethylene oxide group or a propylene oxide group. More preferably, each R₂ is an ethylene oxide group.

Preferably, m is an integer from 1 to 30, more preferably from 2 to 15, and most preferably from 3 to 10.

E is a terminal group comprising one or more of sulfate, phosphate, carboxylate, sulfonate, sulfosuccinate, sulfoacetate, sarcosinate and phosphonate, preferably E comprises sulfate, phosphate or mixtures thereof, more preferably E comprises or is sulfate. M is a solubilizing cation selected from sodium, potassium, ammonium, mono-, di-, tri-alkanolamine and mixtures thereof, preferably M is sodium, potassium or ammonium, more preferably M is sodium or ammonium.

It will be understood that E is a terminal group carrying an anionic charge, covalently bound to the group R₂. M is one or more cationic moieties forming an ionic bond with E to provide charge balance.

Preferably the anionically modified alkyl and/or alkenyl phenol polyoxyalkylene ether is anionically modified alkyl and/or alkenyl phenol polyoxyethylene ether, more preferably is anionically modified cardanol polyoxyethylene ether.

Preferably the anionically modified alkyl and/or alkenyl phenol polyoxyalkylene ether is alkyl and/or alkenyl phenol polyoxyalkylene ether sulfate, or alkyl and/or alkenyl phenol polyoxyalkylene ether phosphate, more preferably, the anionically modified alkyl and/or alkenyl phenol polyoxyalkylene ether is alkyl and/or alkenyl phenol polyoxyethylene ether sulfate, or alkyl and/or alkenyl phenol polyoxyethylene ether phosphate.

It is particularly preferred that the anionically modified alkyl and/or alkenyl phenol polyoxyalkylene ether is cardanol polyoxyalkylene ether sulfate or cardanol polyoxyalkylene ether phosphate, preferably cardanol polyoxyethylene ether sulfate or cardanol polyoxyethylene ether phosphate. Cardanol polyoxyethylene ether sulfate is most preferred.

Cardanol is a product obtained by treating cashew nut shell liquid (CNSL). CNSL is a well-known non-edible natural oil obtained as a by-product of the *Anacardium occidentale* nut. CNSL is one of the most widely used bio-based resource to provide useful chemicals for various applications. Cardanol is an important chemical derived by decarboxylation of anacardic acid, which is the primary component of CNSL. Cardanol is a natural biomass phenol with a C15 side chain (R) in the meta-position of the aromatic ring, which is represented by formula (II). The side chain R is a linear C15 alkyl or alkenyl group comprising 0 to 3 carbon-carbon double bonds. Therefore, cardanol has four components, and each component has saturated, monoene, diene, and triene structures respectively. The positions of double bonds are located at positions 8, 11 and 14 of the side chain R respectively. The four components in cardanol are about 5-8% saturated component, about 48-49% component with one double bond, about 16-17% component with two double bonds and about 29-30% component with three double bonds.

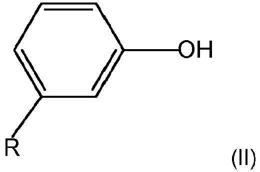

Cardanol polyoxyethylene ether, which is represented by formula (III), may be formed via a polymerization type reaction by reacting cardanol and ethylene oxide in the presence of a catalyst.

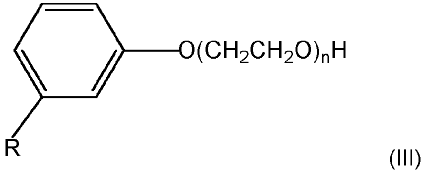

Wherein R is a linear C15 alkyl or alkenyl group comprising 0 to 3 carbon-carbon double bonds as defined above in formula (II). n is an integer from 1 to 50, preferably from 1 to 30, more preferably from 2 to 15, and most preferably from 3 to 10.

The reaction is commonly referred to as ethoxylation. The mechanism of reaction is: cardanol generates oxygen negative ions under alkaline conditions and the cardanol polyoxyethylene ether is obtained by ethoxylation reaction with ethylene oxide. Preferably, the molar ratio of cardanol and ethylene oxide is from 1:100 to 20:1, more preferably from 1:50 to 10:1 and even more preferably from 1:30 to 1:1. Preferred catalysts for this reaction include, for example, potassium hydroxide, sodium hydroxide, barium hydroxide octahydrate, sodium bicarbonate or mixtures thereof. The amount of the catalyst is typically 0.01 to 5% by weight of cardanol, more preferably from 0.1 to 3%, even more preferably from 0.2 to 1% and most preferably from 0.4 to 0.6%. The reaction temperature is preferably from 120 to 180°C and the polymerization reaction time is typically 0.5 to 2 hours. By the end of polymerization reaction, the reaction product is typically neutralized with acetic acid to obtain cardanol polyoxyethylene ether. Other alkylene oxide such as propylene oxide may also react with cardanol through polymerization reaction to produce various cardanol polyoxyalkylene ether. Further examples of manufacturing processes suitable to generate the cardanol polyoxyethylene ether described herein are disclosed in CN102432440A, CN102391080A, CN102351664A and CN101941894A.

The cardanol polyoxyethylene ether may be further functionalized with an anionic group to form anionically modified cardanol polyoxyethylene ether. Preferably the anionic group is sulfate or phosphate, more preferably sulfate.

Cardanol polyoxyethylene ether sulfate, which is represented by formula (IV), may be formed by sulfonating the cardanol polyoxyethylene ether in the presence of a catalyst.

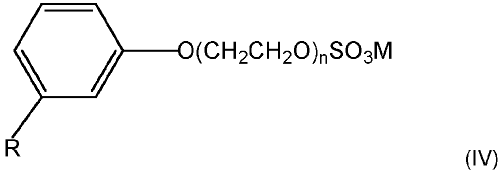

Wherein R is a linear C15 alkyl or alkenyl group comprising 0 to 3 carbon-carbon double bonds as defined above in formula (II). n is an integer from 1 to 50, preferably from 1 to 30, more preferably from 2 to 15, and most preferably from 3 to 10. M is a solubilizing cation selected from sodium, potassium, ammonium, mono-, di-, tri-alkanolamine and mixtures thereof, preferably M is sodium, potassium or ammonium, more preferably M is sodium or ammonium.

Preferred sulfonating agent is sulfamic acid or sodium sulfonate, more preferably sulfamic acid. The molar ratio of cardanol polyoxyethylene ether and sulfonating agent is preferably from 1:10 to 10:1, more preferably from 1:5 to 5:1, even more preferably from 1:3 to 3:1 and most preferably from 1:1.2 to 1:1.

Preferred catalysts for this reaction include, for example, carbonamide, dicyandiamide, urea, p-toluenesulfonic acid, dimethylformamide, N-methylpyrrolidone, hypophosphite or mixtures thereof, more preferably carbonamide, dicyandiamide, urea or mixtures thereof. The molar ratio of cardanol polyoxyethylene ether and the catalyst is preferably from 1:10 to 30:1, more preferably from 1:5 to 20:1, even more preferably from 1:1 to 10:1 and most preferably from 1.5:1 to 5:1. It is preferred that a reducing agent is included in the reaction mixture as a color stabilizer to obtain a light-colored cardanol polyoxyethylene ether sulfate. Preferably, the reducing agent comprises hypophosphorous acid, hypophosphite or mixtures thereof.

The reaction temperature is preferably from 100 to 125°C and the reaction time is typically 3 to 6 hours. By the end of reaction, an alcohol may be added to the reaction mixture to reduce viscosity in order to obtain cardanol polyoxyethylene ether sulfate. The alcohol is preferably a C1-C4 monohydric alcohol comprising methanol, ethanol, isobutanol or mixtures thereof. Alternatively, the reaction mixture may be neutralized with sodium hydroxide to obtain cardanol polyoxyethylene ether sulfate. Further examples of manufacturing processes suitable to generate the cardanol polyoxyethylene ether sulfate described herein are disclosed in CN114276281A and CN101941926A.

When sulfamic acid is used as the sulfonating agent, the counterion M for the obtained cardanol polyoxyethylene ether sulfate is ammonium. An example is commercially available under the trade name NSN3003 from Nasurfar Biomaterial Technology Co. Ltd. The ammonium counterion may be replaced with sodium or potassium ions via ion exchange according to standard processes to produce sodium cardanol polyoxyethylene ether sulfate or potassium cardanol polyoxyethylene ether sulfate. Cardanol polyoxyethylene ether sulfate with a sodium or a potassium counterion is preferred since it may be more stable compared to a corresponding ammonium salt when formulated into a composition of the present invention.

The composition of the present invention preferably comprises the anionically modified alkyl and/or alkenyl phenol polyoxyalkylene ether in an amount of from 0.1 to 30%, more preferably from 0.5 to 20%, even more preferably from 1 to 15% and most preferably from 2 to 10%, based on total weight of the composition and including all ranges subsumed therein.

### Enzyme

The composition of the present invention comprises an enzyme. Examples of enzymes suitable for use in the composition include protease, lipase, amylase, mannanase, pectate lyase, cellulase, phospholipase, cutinase, peroxidase, oxidase or mixtures thereof. Protease and amylase are the most preferred.

Preferably the composition comprises a protease. Protease enzymes hydrolyze bonds within peptides and proteins, in the detergent context this leads to enhanced removal of protein or peptide containing stains. Examples of suitable proteases families include aspartic proteases; cysteine proteases; glutamic proteases; asparagine peptide lyase; serine proteases and threonine proteases. Such protease families are described in the MEROPS peptidase database (http://merops.sanger.ac.uk/). Serine proteases are preferred. Subtilase type serine proteases are more preferred. The term "subtilases" refers to a sub-group of serine protease according to Siezen et al., Protein Engng. 4 (1991) 719-737 and Siezen et al. Protein Science 6 (1997) 501 -523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from Bacillus such as Bacillus lentus, B. alkalophilus, B. subtilis, B. amyloliquefaciens, Bacillus pumilus and Bacillus gibsonii described in; US7262042 and WO09/021867, and subtilisin lentus, subtilisin Novo, subtilisin Carlsberg, Bacillus licheniformis, subtilisin BPN', subtilisin 309, subtilisin 147 and subtilisin 168 described in WO89/06279 and protease PD138 described in (WO93/18140). Preferably the subsilisin is derived from Bacillus, preferably Bacillus lentus, B. alkalophilus, B. subtilis, B. amyloliquefaciens, Bacillus pumilus and Bacillus gibsonii as described in US 6,312,936 B I, US 5,679,630, US 4,760,025, US7,262,042 and WO09/021867. Most preferably the subtilisin is derived from Bacillus gibsonii or Bacillus Lentus.

Suitable commercially available protease enzymes include those sold under the trade names Alcalase^{®}, Blaze^{®}; Duralase^{™}, Durazym^{™}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®}, Esperase^{®} and Carnival^{™} all could be sold as Ultra^{®} or Evity^{®} (Novozymes A/S). Carnival^{™} Evity^{®} is particularly preferred.

Those sold under the tradename Maxatase^{®}, Maxacal^{®}, Maxapem^{®}, Properase^{®}, Purafect^{®}, Purafect Prime^{®}, Purafect Ox^{®}, FN3^{®}, FN4^{®}, Excellase^{®} and Purafect OXP^{®} by Genencor International.

Those sold under the tradename Maxatase^{®}, Maxacal^{®}, Maxapem^{®}, Purafect^{®}, Purafect Prime^{®}, PreferenzTm, Purafect MA^{®}, Purafect Ox^{®}, Purafect OxP^{®}, Puramax^{®}, Properase^{®}, EffectenzTm, FN2^{®}, FN3^{®}, FN4^{®}, Excellase^{®}, Opticlean^{®} and Optimase^{®} (Danisco/DuPont), Axapem^{™} (Gist-Brocases N.V.),

Those available from Henkel/ Kemira, namely BLAP (sequence shown in Figure 29 of US 5,352,604 with the following mutations S99D + SIOI R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) - all from Henkel/Kemira; and KAP (Bacillus alkalophilus subtilisin with mutations A230V + S256G + S259N) from Kao.

Preferably the composition comprises an amylase. Amylases are enzymes that catalyze the hydrolysis of starch into sugars, in the detergent context this leads to enhanced removal of starch containing stains. Suitable amylases (alpha and/or beta) include those of bacterial or fungal origin. Chemically modified or protein engineered mutants are included. More preferred amylases include, for example, alpha-amylases obtained from Bacillus, e.g. a special strain of *B. licheniformis,* described in more detail in GB 1,296,839, or the Bacillus sp. strains disclosed in WO 95/026397 or WO 00/060060. Even more preferred (commercially available) amylases are sold under the tradenames Duramyl^{®}, Termamyl^{®}, Fungamyl^{®}, Stainzyme^{®}, Stainzyme^{®} Plus, Natalase^{®}, Amplify Prime^{®} and BAN^{®} (from Novozymes A/S), and Rapidase^{®}, Purastar^{®}/Effectenz^{™}, Powerase^{™}, Preferenz S1000^{™} Preferenz S1 10^{™} and Preferenz S100^{™} (from Genencor International Inc./DuPont). The Amplify^{®} Prime amylase is most advantageous of the commercial amylase enzyme.

Lipase can be any known lipase used in the art of detergent compositions. Preferred are lipases from *Humicola* (synonym *Thermomyces),* e.g. from other *H. lanuginosa (T. lanuginosus)* strains or from *H. insolens,* a Pseudomonas lipase, e.g. from *P. alcaligenes* or *P. pseudoalcaligenes, P. cepacia, P. stutzeri, P. fluorescens, Pseudomonas sp.* strain SD 705 (WO 95/06720 and WO 96/27002), *P. wisconsinensis,* a Bacillus lipase, e.g. from *B. subtilis* (Dartois et al. (1993), Biochemica et Biophysica Acta, 1131, 253-360), *B. stearothermophilus* (JP 64/744992) or B. *pumilus* (WO 91/16422). Even more preferred (commercially available) lipases are sold under the tradenames Lipex^{®}, Lipolase^{®} and Lipolase Ultra^{®}, Lipoprime^{®} and Lipoclean^{®} (from Novozymes A/S), and the Bacterial enzyme, Lipomax^{®} ex Genecor. This is a bacterially derived Lipase, of variant M21L of the lipase of Pseudomonas alcaligenes as described in WO 94/25578 to Gist-Brocades (M.M.M.J. Cox, H.B.M. Lenting, L.J.S.M. Mulleners and J.M. van der Laan). The Lipex^{®} Lipase is most preferred.

Suitable mannanases include mannanases of bacterial and fungal origin. More preferred are mannases derived from filamentous fungus genus Aspergillus, preferably *Aspergillus niger* or *Aspergillus aculeatus* (WO 94/25576); *Trichoderma reseei* (as disclosed in WO 93/24622); Bacillus organisms (e.g. as described in Talbot et al., Appl. Environ. Microbiol. Vol.56, No. 11, pp. 3505-3510 (1990), which describes a beta-mannanase derived from *Bacillus stearothermophilus,* Mendoza et al., World J. Microbiol. Biotech., Vol. 10, No. 5, pp. 551-555 (1994), which describes a beta-mannanase derived from *Bacillus subtilis,* JP-A-03047076 which describes a beta-mannanase derived from Bacillus sp., JP-A-63056289 which describes the production of an alkaline, thermostable beta-mannanase, JP-A-63036775 which describes Bacillus microorganism FERM P-8856 which produces beta-mannanase and beta-mannosidase, JP-A-08051975 which describes a alkaline beta-mannanases from alkalophilic Bacillus sp. AM-001, WO97/11164 which described a purified mannanase from *Bacillus amyloliquefaciens,* WO 91/18974 which describes a hemicellulase such as a glucanase, xylanase or mannanase active). Also preferred are the alkaline family 5 and 26 mannanases derived from *Bacillus agaradhaerens, Bacillus licheniformis, Bacillus halodurans, Bacillus clausii,* Bacillus sp., and *Humicola insolens* (as disclosed in WO 99/64619). More preferred bacterial mannases are those described in WO 99/64619. Even more preferred (commercially available) mannanase is Mannaway^{®} available from Novozymes A/S Denmark.

Pectate lyases are also called polygalacturonate lyases. Preferred are pectate lyases that have been derived from bacterial genera such as *Erwinia, Pseudomonas, Klebsiella* and *Xanthomonas, Bacillus.* More preferred are pectate lyases obtained from *Bacillus subtilis* (Nasser et al. (1993) FEBS Letts. 335:319-326), *Bacillus sp. YA-14* (Kim et al. (1994) Biosci. Biotech. Biochem. 58:947-949); *Bacillus pumilus* (Dave and Vaughn (1971) J. Bacteriol. 108:166-174), *B. polymyxa* (Nagel and Vaughn (1961) Arch. Biochem. Biophys. 93:344-352), B. *stearothermophilus* (Karbassi and Vaughn (1980) Can. J. Microbiol. 26:377-384), *Bacillus sp.* (Hasegawa and Nagel (1966) J. Food Sci. 31:838-845), *Bacillus sp. RK9* (Kelly and Fogarty (1978) Can. J. Microbiol. 24:1164-1172), as disclosed in Heffron et al., (1995) Mol. Plant-Microbe Interact. 8: 331-334, Henrissat et al., (1995) Plant Physiol. 107: 963-976, as disclosed in WO 99/27083, WO 99/27084, US6,284,524, WO 02/006442 (in particular as disclosed in the Examples).

Even more preferred are (commercially available) pectate lyases are BioPrep^{®}, Scourzyme^{®} L, and XPec^{®} from Novozymes A/S, Denmark. The XPec^{®} pectate lyase is most preferred.

Suitable cellulase include those of bacterial, fungal, insect and/or mammalian origin. Chemically modified or protein engineered mutants are included. More preferred are cellulases from the genera *Bacillus, Pseudomonas, Humicola, Fusarium, Thielavia, Acremonium,* e.g. the fungal cellulases produced from *Humicola insolens, Thielavia terrestris, Myceliophthora thermophila,* and *Fusarium oxysporum* disclosed in US 4,435,307, US 5,648,263, US 5,691,178, US 5,776,757, WO 89/09259, WO 96/029397, and WO 98/012307. Even more preferred (commercially available) cellulases are Celluzyme^{®}, Carezyme^{®}, Endolase^{™}, Renozyme^{®}, Celluclean^{®} (Novozymes A/S), Clazinase^{™} and Puradax HA^{™} (Genencor International Inc.), and KAC-500(B)^{™} (Kao Corporation). The Celluclean^{®} cellulase is most preferred.

Phospholipase are classified as EC 3.1.1.4 and/or EC 3.1.1.32. As used herein, the term phospholipase is an enzyme, which has activity towards phospholipids. Phospholipids, such as lecithin or phosphatidylcholine, consist of glycerol esterified with two fatty acids in an outer (sn-1) and the middle (sn-2) positions and esterified with phosphoric acid in the third position; the phosphoric acid, in turn, may be esterified to an amino-alcohol. Phospholipases are enzymes which participate in the hydrolysis of phospholipids. Several types of phospholipase activity can be distinguished, including phospholipases A1 and A2 which hydrolyze one fatty acyl group (in the sn-1 and sn-2 position, respectively) to form lysophospholipid; and lysophospholipase (or phospholipase B) which can hydrolyze the remaining fatty acyl group in lysophospholipid. Phospholipase C and phospholipase D (phosphodiesterases) release diacyl glycerol or phosphatidic acid respectively.

Cutinases are classified in EC 3.1.1.74. The cutinase used according to the invention may be of any origin. Preferably the cutinases are of microbial origin and more preferably of bacterial, of fungal or of yeast origin.

Suitable peroxidases/oxidases are of bacterial, fungal or mammalian origin and more preferably of bacterial origin. Chemically modified or protein engineered mutants are included. Preferably the peroxidases/oxidases are derived from *Aeromonas sp.*

The composition of the present invention preferably comprises from 0.00001 to 1% by weight of the enzyme, more preferably from 0.0001 to 0.5%, even more preferably from 0.0005 to 0.4%, still even more preferably from 0.001 to 0.3% and most preferably from 0.001 to 0.2%, based on total weight of the composition and including all ranges subsumed therein. Amounts of wt. % enzymes in the composition refer to wt. % of active protein levels.

Enzymes may be added in liquid, granular or in encapsulated form to the composition, but preferably are not encapsulated. The composition may also comprise enzyme stabilizers e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

### Surfactant

The composition may comprise other surfactants in addition to the anionically modified alkyl and/or alkenyl phenol polyoxyalkylene ether. Suitable surfactants comprise anionic surfactants, non-ionic surfactants, cationic surfactants, amphoteric surfactant or mixtures thereof, preferably the surfactants comprise anionic surfactants, non-ionic surfactants or mixtures thereof.

A preferred class of anionic surfactant may be used in the invention includes alkylbenzene sulfonates, particularly linear alkylbenzene sulfonates (LAS) with an alkyl chain length of from 10 to 18 carbon atoms. Commercial LAS is a mixture of closely related isomers and homologues alkyl chain homologues, each containing an aromatic ring sulfonated at the *"para"* position and attached to a linear alkyl chain at any position except the terminal carbons. The linear alkyl chain typically has a chain length of from 11 to 15 carbon atoms, with the predominant materials having a chain length of about C12. Each alkyl chain homologue consists of a mixture of all the possible sulfophenyl isomers except for the 1-phenyl isomer. LAS is normally formulated into compositions in acid (i.e. HLAS) form and then at least partially neutralized *in-situ.* Examples of alkylbenzene sulfonates include sodium salt of linear alkylbenzene sulphonate, alkyl toluene sulphonate, alkyl xylene sulphonate, alkyl phenol sulphonate, alkyl naphthalene-sulphonate, ammonium diamylnaphthalene-sulphonate and sodium dinonylnaphthalene-sulphonate and mixtures with olefin sulphonates.

Some alkyl sulfate surfactant (PAS) may be used, such as non-ethoxylated primary and secondary alkyl sulphates with an alkyl chain length of from 10 to 18.

Another anionic surfactant commonly used in compositions are alkyl ether sulfates having a straight or branched chain alkyl group having 10 to 18, more preferably 12 to 14 carbon atoms and containing an average of 1 to 3EO units per molecule. A preferred example is sodium lauryl ether sulfate (SLES) in which the predominantly C₁₂ lauryl alkyl group has been ethoxylated with an average of 2EO units per molecule.

Alkyl ether sulfates may be present in the composition. Preferably, the composition is substantially free of alkyl ether sulfates. "Substantially free of", as used herein, means less than 1.5%, preferably less than 1.0%, more preferably less than 0.75%, more preferably still less than 0.5% and even more preferably less than 0.1% and most preferably from 0 to 0.01% by weight, based on total weight of the composition, including all ranges subsumed therein. It is preferred that the composition does not comprise any alkyl ether sulfates.

When the composition comprises other anionic surfactants in addition to the anionically modified alkyl and/or alkenyl phenol polyoxyalkylene ether, the amount of total anionic surfactants in the composition preferably ranges from 0.1 to 60%, more preferably from 1 to 55% and even more preferably from 3 to 50%, based on total weight of the composition and including all ranges subsumed therein.

The composition may also comprise non-ionic surfactants. Non-ionic surfactants for use in the invention include, for example, a) polyoxyalkylene compounds, i.e. the reaction product of alkylene oxides (such as ethylene oxide or propylene oxide or mixtures thereof) with starter molecules having a hydrophobic group and a reactive hydrogen atom which is reactive with the alkylene oxide. Such starter molecules include alcohols, acids, amides or alkyl phenols. Where the starter molecule is an alcohol, the reaction product is known as an alcohol alkoxylate. The polyoxyalkylene compounds can have a variety of block and heteric (random) structures. For example, they can comprise a single block of alkylene oxide, or they can be diblock alkoxylates or triblock alkoxylates. Within the block structures, the blocks can be all ethylene oxide or all propylene oxide, or the blocks can contain a heteric mixture of alkylene oxides. Examples of such materials include C₈ to C₂₂ alkyl phenol ethoxylates with an average of from 5 to 25 moles of ethylene oxide per mole of alkyl phenol; and alkyl alcohol ethoxylates such as C₈ to C₁₈ primary or secondary linear or branched alcohol ethoxylates with an average of from 2 to 40 moles of ethylene oxide per mole of alcohol; b) fatty acid amides; c) alkoxylated glycerol esters; d) alkyl poly glycosides; e) rhamnolipids; or a mixture thereof.

A preferred class of non-ionic surfactant for use in the present invention includes C₈ to C₁₈ alkyl alcohol ethoxylates, more preferably C₁₂ to C₁₅ primary linear alcohol ethoxylates with an average of from 3 to 20, more preferably from 3 to 10 moles of ethylene oxide per mole of alcohol. Particularly preferred are lauryl alcohol condensed with 3, 5, 7 and 9 moles of EO (AEO-3, AEO-5, AEO-7 and AEO-9).

Another preferred class of non-ionic surfactant for use in the invention includes fatty acid amides. Preferably, the fatty acid amide contains at least 6 carbon atoms. Suitable fatty acid preferably contains from 8 to 24 carbon atoms. preferably from 12 to 20 carbon atoms, and most preferably from 12 to 18 carbon atoms. In the most preferred embodiment of the invention, amides of essential fatty acids are employed. Amides suitable for use in the present invention may be simple amides (i.e., those containing a -CONH₂ group), N-alkyl amides, N, N-dialkyl amides, mono-alkanol amides, and di-alkanol amides. Suitable alkyl or alkanol groups contain from 1 to 30 carbon atoms, preferably from 1 to 20 carbon atoms, and most preferably from 1 to 8 carbon atoms. The preferred amides included in the present invention are mono- and di-alkanol amides, particularly of essential fatty acids. Alkanol amides are more commonly available than alkyl amides.

Preferably, the fatty acid amide is fatty alkanolamides (fatty acid alkanolamides), more preferably C₈ to C₂₀ fatty acid C₁ to C₈ alkanolamide. The preferred fatty acid amides are selected from mono- and diethanolamides of linoleic acid, palmitic acid, and coconut oil. More preferably the fatty acid amide comprises cocamide MEA, cocamide DEA, lauramide DEA, palm kernelamide DEA, stearamide MEA, myristamide DEA, stearamide DEA, oleylamide DEA, tallowamide DEA, tallowamide MEA, isostearamide DEA, isostearamide MEA, cocamide MIPA, or a mixture thereof. Palm kernelamide DEA is particularly preferred.

Another preferred class of non-ionic surfactant is alkoxylated glycerol esters. The alkoxylated glycerol ester is represented by the formula (V):

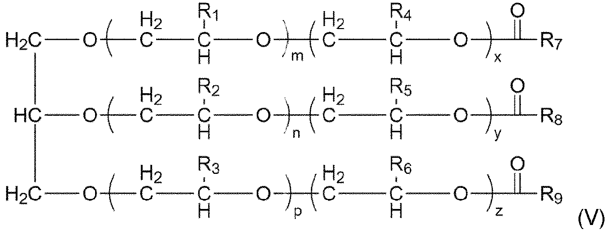

Wherein each of R₁ to R₆ is independently a hydrogen or a methyl group; each of R₇ to R₉ is independently a linear or branched, alkyl or alkenyl group having 5 to 30 carbon atoms, preferably from 8 to 22 carbon atoms more preferably from 10 to 18 carbon atoms; m, n, p, x, y, or z is independently a number of from 1 to 30, preferably from 5 to 25 and more preferably from 12 to 21. The sum of m, n, p, x, y, z being in the range of 3 to 90.

Preferably, the alkoxylated glycerol ester comprises coconut fatty acid esters. Coconut or coco fatty acids include around 82%wt. saturated fatty acids and of the total fatty acid content lauric acid is the most common at around 48% wt. of the fatty acid content. Myristic acid (16%wt.) and palmitic acid (9.5%wt.) are the next most common. Oleic acid is the most common unsaturated acid present at around 6.5% wt. of the fatty acid content.

Preferably, the alkoxylated glycerol ester comprises palm oil fatty acid esters. Palm oil has a balanced fatty acid composition in which the level of saturated fatty acids is almost equal to that of the unsaturated fatty acids. Palmitic acid (44%-45%) and oleic acid (39%-40%) are the major component acids, with linoleic acid (10%-11%) and only a trace amount of linolenic acid. Palm kernel oil contains more saturated fatty acids compared to palm oil. The major fatty acids in palm kernel oil are about 48% lauric acid, 16% myristic acid and 15% oleic acid. The most preferred alkoxylated glycerol ester is palm kernel oil ethoxylates. An example is commercially available under the trade name SOE-N-60 from Sinolight Surfactant Technology Co., Ltd.

Other suitable alkoxylated glyceryl esters are commercially available from Kao under the Levenol brand name. Variants such as Levenol F-200 which has an average EO of 6 and a molar ratio between glycerol and coco fatty acid of 0.55, Levenol V501/2 which has an average EO of 17 and a molar ratio between glycerol and coco fatty acid of 1.5 and Levenol C201 which is also known as glycereth-17 cocoate.

Another class of non-ionic surfactants which can be used in accordance with this invention are glycoside surfactants. Alkyl poly glycoside surfactants suitable for use in accordance with the present invention include those of the formula:

RO-(R²O)_{y}-(Z)ₓ

wherein R is a monovalent organic radical containing from about 6 to about 30 (preferably from about 8 to about 18) carbon atoms; R² is a divalent hydrocarbon radical containing from about 2 to 4 carbons atoms; O is an oxygen atom; y is a number which can have an average value of from 0 to about 12 but which is most preferably zero; Z is a moiety derived from a reducing saccharide containing 5 or 6 carbon atoms; and x is a number having an average value of from 1 to about 10 (preferably from about 1 1/2 to about 10).

A particularly preferred group of glycoside surfactants for use in the practice of this invention includes those of the formula above in which R is a monovalent organic radical (linear or branched) containing from about 6 to about 18 (especially from about 8 to about 18) carbon atoms; y is zero; z is glucose or a moiety derived therefrom; x is a number having an average value of from 1 to about 4 (preferably from about 1 1/2 to 4).

Preferably, the non-ionic surfactant comprises alkyl alcohol ethoxylates. Mixtures of two or more of the non-ionic surfactants can be used. When the composition comprises non-ionic surfactants, the non-ionic surfactant is typically present at a level from 0.01 to 30%, more preferably from 0.1 to 20% and most preferably from 1 to 10%, based on total weight of the composition and including all ranges subsumed therein.

The composition may also comprise one or more types of cationic surfactant. Many cationic surfactants are known in the art, and almost any cationic surfactant having at least one long chain alkyl group of about 10 to 24 carbon atoms may be present as an auxiliary component of the surfactant system. Such compounds are described in "Cationic Surfactants", Jungermann, 1970.

Specific cationic surfactants include C8 to C18 alkyl dimethyl ammonium halides and derivatives thereof in which one or two hydroxyethyl groups replace one or two of the methyl groups, and mixtures thereof. More cationic surfactants which can be used as surfactants are described in detail in U.S. Patent No. 4,497,718.

As with the non-ionic and anionic surfactants, the compositions of the invention may use cationic surfactants alone or in combination with any of the other surfactants known in the art. Cationic surfactant, when included, may be present in an amount ranging from 0 to 5% based on total weight of the composition. It is preferred that the composition does not comprise any cationic surfactants.

The composition may also comprise one or more types of amphoteric surfactant. Specific amphoteric (zwitterionic) surfactants include alkyl amine oxides, alkyl betaines, alkyl amidopropyl betaines, alkyl sulfobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, having alkyl radicals containing from about 8 to about 22 carbon atoms, the term "alkyl" being used to include the alkyl portion of higher acyl radicals. Amphoteric (zwitterionic) surfactant, when included, may be present in an amount ranging from 0 to 5% based on total weight of the composition. It is preferred that the composition does not comprise any amphoteric surfactants.

### Builders

Builders enhance or maintain the cleaning efficiency of the surfactant, primarily by reducing water hardness. This is done either by sequestration or chelation (holding hardness minerals in solution), by precipitation (forming an insoluble substance), or by ion exchange (trading electrically charged particles). Suitable builders can be of the organic or inorganic type, or a mixture thereof.

Suitable inorganic builders include hydroxides, carbonates, sesquicarbonates, bicarbonates, silicates, zeolites, and mixtures thereof. Specific examples of such materials include sodium and potassium hydroxide, sodium and potassium carbonate, sodium and potassium bicarbonate, sodium sesquicarbonate, sodium silicate and mixtures thereof.

Suitable organic builders include polycarboxylates, in acid and/or salt form. When utilized in salt form, alkali metal (e.g. sodium and potassium) or alkanolammonium salts are preferred. Specific examples of such materials include sodium and potassium citrates, sodium and potassium tartrates, the sodium and potassium salts of tartaric acid monosuccinate, the sodium and potassium salts of tartaric acid disuccinate, sodium and potassium ethylenediaminetetraacetates, sodium and potassium N(2-hydroxyethyl)-ethylenediamine triacetates, sodium and potassium nitrilotriacetates and sodium and potassium N-(2-hydroxyethyl)-nitrilodiacetates. Polymeric polycarboxylates may also be used, such as polymers of unsaturated monocarboxylic acids (e.g. acrylic, methacrylic, vinylacetic, and crotonic acids) and/or unsaturated dicarboxylic acids (e.g. maleic, fumaric, itaconic, mesaconic and citraconic acids and their anhydrides). Specific examples of such materials include polyacrylic acid, polymaleic acid, and copolymers of acrylic and maleic acid. The polymers may be in acid, salt or partially neutralised form and may suitably have a molecular weight (Mw) ranging from about 1,000 to 100,000, preferably from about 2,000 to about 85,000, and more preferably from about 2,500 to about 75,000.

It is particularly preferred that the builders are phosphate sequestrants. Examples of phosphate sequestrants suitable for use in the composition include 1-hydroxyethylidene-1,1-diphosphonic acid (HEDP), diethylenetriaminepenta(methylenephosphonic acid) (DTPMP), hexamethylenediaminetetra(methylenephosphonic acid) (HDTMP), aminotris(methylenephosphonic acid) (ATMP), ethylenediaminetetra(methylenephosphonic acid) (EDTMP), tetramethylenediaminetetra(methylenephosphonic acid) (TDTMP), phosphonobutanetricarboxylic acid (PBTC) or mixtures thereof, preferably 1-hydroxyethylidene-1,1-diphosphonic acid (HEDP), diethylenetriaminepenta(methylenephosphonic acid) (DTPMP) or mixtures thereof. Diethylenetriaminepenta(methylenephosphonic acid) (DTPMP) is particularly preferred.

The sequestrant can be in the form of an acid or a corresponding salt. Preferably the sequestrant is in the form of a corresponding salt, more preferably an alkali metal salt and even more preferably a sodium salt.

Mixtures of any of the above described materials may also be used.

The composition of the present invention preferably comprises the builders in an amount of from 0.01 to 10%, more preferably from 0.1 to 5%, even more preferably from 0.25 to 4% and most preferably from 0.5 to 2.5%, based on total weight of the composition and including all ranges subsumed therein.

### Hydrotropes

A composition of the invention preferably comprises non-aqueous carriers such as hydrotropes, co-solvents and phase stabilizers. Such materials are typically low molecular weight, water-soluble or water-miscible organic liquids such as C1 to C5 monohydric alcohols (such as ethanol and n- or i-propanol); C2 to C6 diols (such as monopropylene glycol and dipropylene glycol); C3 to C9 triols (such as glycerol); polyethylene glycols having a weight average molecular weight (M_{w}) ranging from about 200 to 600; C1 to C3 alkanolamines such as mono-, di- and triethanolamines; and alkyl aryl sulfonates having up to 3 carbon atoms in the lower alkyl group (such as the sodium and potassium xylene, toluene, ethylbenzene and isopropyl benzene (cumene) sulfonates).

Mixtures of any of the above described materials may also be used.

Non-aqueous carriers, when included, may be present in an amount ranging from 0.01 to 50% by weight of the composition, preferably from 0.05 to 30%, more preferably from 0.1 to 15% and even more preferably from 0.2 to 5%, based on total weight of the composition and including all ranges subsumed therein. The level of hydrotrope used is linked to the level of surfactant and it is desirable to use hydrotrope level to manage the viscosity in such compositions. The preferred hydrotrope are monopropylene glycol, glycerol, triethanolamines or mixtures thereof.

### Soil Release Polymers

Soil release polymers (SRP) help to improve the detachment of soils from fabric by modifying the fabric surface during washing. The adsorption of a SRP over the fabric surface is promoted by an affinity between the chemical structure of the SRP and the target fibre.

The composition of the invention preferably comprises SRPs. SRPs for use in the invention may include a variety of charged (e.g. anionic) as well as non-charged monomer units and structures may be linear, branched or star-shaped. The SRP structure may also include capping groups to control molecular weight or to alter polymer properties such as surface activity. The weight average molecular weight (M_{w}) of the SRP may suitably range from about 1000 to about 20,000 and preferably ranges from about 1500 to about 10,000.

SRPs for use in the invention may suitably be selected from copolyesters of dicarboxylic acids (for example adipic acid, phthalic acid or terephthalic acid), diols (for example ethylene glycol or propylene glycol) and polydiols (for example polyethylene glycol or polypropylene glycol). The copolyester may also include monomeric units substituted with anionic groups, such as for example sulfonated isophthaloyl units. Examples of such materials include oligomeric esters produced by transesterification/oligomerization of poly(ethyleneglycol) methyl ether, dimethyl terephthalate ("DMT"), propylene glycol ("PG") and poly(ethyleneglycol) ("PEG"); partly- and fully-anionic-end-capped oligomeric esters such as oligomers from ethylene glycol ("EG"), PG, DMT and Na-3,6-dioxa-8-hydroxyoctanesulfonate; nonionic-capped block polyester oligomeric compounds such as those produced from DMT, Me-capped PEG and EG and/or PG, or a combination of DMT, EG and/or PG, Me-capped PEG and Na-dimethyl-5-sulfoisophthalate, and copolymeric blocks of ethylene terephthalate or propylene terephthalate with polyethylene oxide or polypropylene oxide terephthalate.

Other types of SRP for use in the invention include cellulosic derivatives such as hydroxyether cellulosic polymers, C₁-C₄ alkylcelluloses and C₄ hydroxyalkyl celluloses; polymers with poly(vinyl ester) hydrophobic segments such as graft copolymers of poly(vinyl ester), for example C₁-C₆vinyl esters (such as poly(vinyl acetate)) grafted onto polyalkylene oxide backbones; poly(vinyl caprolactam) and related co-polymers with monomers such as vinyl pyrrolidone and/or dimethylaminoethyl methacrylate; and polyester-polyamide polymers prepared by condensing adipic acid, caprolactam, and polyethylene glycol.

Preferred SRPs for use in the invention include copolyesters formed by condensation of terephthalic acid ester and diol, preferably 1,2 propanediol, and further comprising an end cap formed from repeat units of alkylene oxide capped with an alkyl group. Examples of such materials have a structure corresponding to general formula (VI): 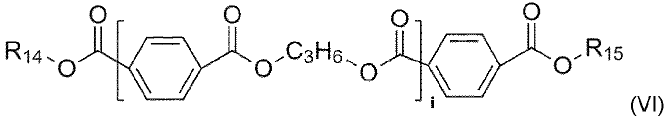
in which R₁₄ and R₁₅ independently of one another are X-(OC₂H₄),-(OC₃H₆)_{;}
in which X is C₁₋₄ alkyl and preferably methyl;
q is a number from 12 to 120, preferably from 40 to 50;
s is a number from 1 to 10, preferably from 1 to 7; and
i is a number from 4 to 9.

Because they are averages, q, s and i are not necessarily whole numbers for the polymer in bulk.

Mixtures of any of the above described materials may also be used.

The overall level of SRP, when included, may range from 0.1 to 10% by weight of the composition, depending on the level of polymer intended for use in the final composition and which is desirably from 0.3 to 7%, more preferably from 0.5 to 5%, based on total weight of the composition and including all ranges subsumed therein.

Suitable SRPs are described in greater detail in U. S. Patent Nos. 5,574,179; 4,956,447; 4,861,512; 4,702,857, WO 2007/079850 and WO2016/005271. If employed, SRPs will typically be incorporated into the composition herein in concentrations ranging from 0.01 to 10%, more preferably from 0.1 to 5% by weight of the composition.

### Polymeric Cleaning Boosters

To further improve the environmental profile of the composition, it may be preferred in some cases to reduce the volume of composition dosed per wash-load and to add various highly weight efficient ingredients to the composition to boost cleaning performance. In addition to the soil release polymers of the invention described above, a composition of the invention will preferably contain one or more additional polymeric cleaning boosters such as anti-redeposition polymers.

Anti-redeposition polymers stabilise the soil in the wash solution thus preventing redeposition of the soil. Suitable anti-redeposition polymers for use in the invention include alkoxylated polyethyleneimines. Polyethyleneimines are materials composed of ethylene imine units - CH₂CH₂NH- and, where branched, the hydrogen on the nitrogen is replaced by another chain of ethylene imine units. Preferred alkoxylated polyethyleneimines for use in the invention have a polyethyleneimine backbone of about 300 to about 10000 weight average molecular weight (M_{w}). The polyethyleneimine backbone may be linear or branched. It may be branched to the extent that it is a dendrimer. The alkoxylation may typically be ethoxylation or propoxylation, or a mixture of both. Where a nitrogen atom is alkoxylated, a preferred average degree of alkoxylation is from 10 to 30, preferably from 15 to 25 alkoxy groups per modification. A preferred material is ethoxylated polyethyleneimine, with an average degree of ethoxylation being from 10 to 30, preferably from 15 to 25 ethoxy groups per ethoxylated nitrogen atom in the polyethyleneimine backbone.

Mixtures of any of the above described materials may also be used.

When included, a composition of the invention will preferably comprise from 0.025 to 8% by weight of one or more anti-redeposition polymers such as, for example, the alkoxylated polyethyleneimines which are described above.

### Preservative

The composition preferably comprises a preservative or a mixture of preservatives. Preferably the preservative is selected from benzoic acid and salts thereof, alkylesters of p-hydroxybenzoic acid and salts thereof, sorbic acid, diethyl pyrocarbonate, dimethyl pyrocarbonate, preferably benzoic acid and salts thereof, most preferably sodium benzoate. The preservative is preferably present in an amount from 0.01 to 3% by weight of the composition, preferably from 0.3% to 1.5%. Weights are calculated for the protonated form.

### Fluorescent Agent

It may be advantageous to include fluorescent agents (optical brightener) in the compositions. Usually, these fluorescent agents are supplied and used in the form of their alkali metal salts, for example, the sodium salts. The total amount of the fluorescent agent or agents used in the composition is generally from 0.005 to 2%, more preferably 0.01 to 0.5% by weight of the composition.

Preferred classes of fluorescent agents are: Di-styryl biphenyl compounds, e.g. Tinopal (Trade Mark) CBS-X, Di-amine stilbene di-sulphonic acid compounds, e.g. Tinopal DMS pure Xtra, Tinopal 5BMGX, and Blankophor (Trade Mark) HRH, and Pyrazoline compounds, e.g. Blankophor SN.

Preferred fluorescent agents are: sodium 2 (4-styryl-3-sulfophenyl)-2H-napthol[1,2-d]triazole, disodium 4,4'-bis{[(4-anilino-6-(N methyl-N-2 hydroxyethyl) amino 1,3,5-triazin-2-yl)]amino}stilbene-2-2' disulfonate, disodium 4,4'-bis{[(4-anilino-6-morpholino-1,3,5-triazin-2-yl)]amino} stilbene-2-2' disulfonate, and disodium 4,4'-bis(2-sulfoslyryl)biphenyl. Most preferably the fluoescer is a di-styryl biphenyl compound, preferably sodium 2,2'-([1,1'-biphenyl]-4,4'-diylbis(ethene-2,1-diyl))dibenzenesulfonate (CAS-No 27344-41-8).

### Anti-foam

The composition may also comprise an anti-foam. Anti-foam materials are well known in the art and include silicones, fatty acids, fatty alcohols and EO-PO block copolymers.

Preferably, where present, the fatty acid anti-foam is present at from 1.3 to 3.0% by weight of the composition, more preferably from 1.4 to 2.0% and most preferably from 1.6 to 1.65%.

Suitable fatty acids in the context of this invention include aliphatic carboxylic acids of formula R¹²COOH, where R¹² is a linear or branched alkyl or alkenyl chain containing from 6 to 24, more preferably 10 to 22, most preferably from 12 to 18 carbon atoms and 0 or 1 double bond. Preferred examples of such materials include saturated C12-18 fatty acids such as lauric acid, myristic acid, palmitic acid or stearic acid; and fatty acid mixtures in which 50 to 100% (by weight based on the total weight of the mixture) consists of saturated C12-18 fatty acids. Such mixtures may typically be derived from natural fats and/or optionally hydrogenated natural oils (such as coconut oil, palm kernel oil or tallow).

The fatty acids may be present in the form of their sodium, potassium or ammonium salts and/or in the form of soluble salts of organic bases, such as mono-, di- or triethanolamine. Suitable fatty alcohols in the context of this invention include aliphatic alcohol of formula R¹³OH, where R¹³ is a linear or branched alkyl or alkenyl chain containing from 6 to 24, more preferably 10 to 22, most preferably from 12 to 18 carbon atoms.

Suitable EO-PO block copolymers in the context of this invention include a polymer with repeating units of ethylene oxide and propylene oxide and with hydrophile lipophile balance (HLB) value equal or smaller than 4.

Mixtures of any of the above described materials may also be used.

For formula accounting purposes, in the formulation, fatty acids and/or their salts (as defined above) are not included in the level of surfactant or in the level of builder.

### Shading dyes

Shading dye may be used to improve the performance of the compositions. Preferred dyes are violet or blue. It is believed that the deposition on fabrics of a low level of a dye of these shades, masks yellowing of fabrics. A further advantage of shading dyes is that they can be used to mask any yellow tint in the composition itself.

Shading dyes are well known in the art of laundry liquid formulation.

Suitable and preferred classes of dyes are discussed below.

### Direct Dyes:

Direct dyes (otherwise known as substantive dyes) are the class of water soluble dyes which have an affinity for fibres and are taken up directly. Direct violet and direct blue dyes are preferred.

Preferably bis-azo or tris-azo dyes are used.

Most preferably, the direct dye is a direct violet of the following structures: 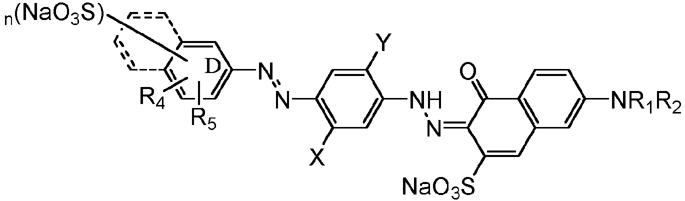 or 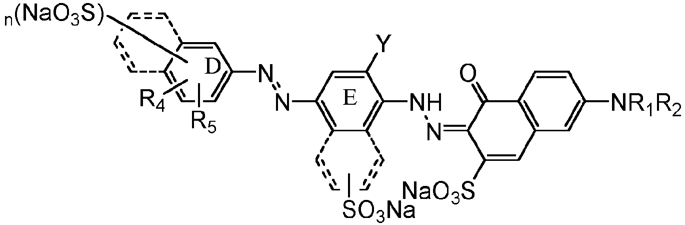 wherein:
ring D and E may be independently naphthyl or phenyl as shown;
R₁ is selected from: hydrogen and C₁-C₄-alkyl, preferably hydrogen;
R₂ is selected from: hydrogen, C₁-C₄-alkyl, substituted or unsubstituted phenyl and substituted or unsubstituted naphthyl, preferably phenyl;
R₃ and R₄ are independently selected from: hydrogen and C₁-C₄-alkyl, preferably hydrogen or methyl;
X and Y are independently selected from: hydrogen, C₁-C₄-alkyl and C₁-C₄-alkoxy; preferably the dye has X= methyl; and, Y = methoxy and n is 0, 1 or 2, preferably 1 or 2.

Preferred dyes are direct violet 7, direct violet 9, direct violet 11, direct violet 26, direct violet 31, direct violet 35, direct violet 40, direct violet 41, direct violet 51, and direct violet 99. Bis-azo copper containing dyes for example direct violet 66 may be used. The benzidene based dyes are less preferred.

Preferably the direct dye is present at 0.000001 to 1%, more preferably 0.00001% to 0.0010% by weight of the composition.

In another embodiment the direct dye may be covalently linked to the photo-bleach, for example as described in WO2006/024612.

### Acid dyes:

Cotton substantive acid dyes give benefits to cotton containing garments. Preferred dyes and mixes of dyes are blue or violet. Preferred acid dyes are:
(i) azine dyes, wherein the dye is of the following core structure: 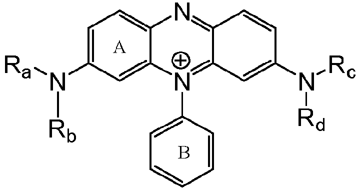
   wherein Rₐ, R_{b}, R_{c} and R_{d} are selected from: H, a branched or linear C₁ to C₇-alkyl chain, benzyl a phenyl, and a naphthyl;
   the dye is substituted with at least one SO₃⁻ or -COO⁻ group;
   the B ring does not carry a negatively charged group or salt thereof; and
   the A ring may further substituted to form a naphthyl; the dye is optionally substituted by groups selected from: amine, methyl, ethyl, hydroxyl, methoxy, ethoxy, phenoxy, Cl, Br, I, F, and NO₂.

Preferred azine dyes are: acid blue 98, acid violet 50, and acid blue 59, more preferably acid violet 50 and acid blue 98.

Other preferred non-azine acid dyes are acid violet 17, acid black 1 and acid blue 29.

Preferably the acid dye is present at 0.0005% to 0.01% by weight of the composition.

### Hydrophobic dyes:

The composition may comprise one or more hydrophobic dyes selected from benzodifuranes, methine, triphenylmethanes, napthalimides, pyrazole, napthoquinone, anthraquinone and mono-azo or di-azo dye chromophores. Hydrophobic dyes are dyes which do not contain any charged water solubilising group. Hydrophobic dyes may be selected from the groups of disperse and solvent dyes. Blue and violet anthraquinone and mono-azo dye are preferred.

Preferred dyes include solvent violet 13, disperse violet 27 disperse violet 26, disperse violet 28, disperse violet 63 and disperse violet 77.

Preferably the hydrophobic dye is present at 0.0001% to 0.005% by weight of the composition.

### Basic dyes:

Basic dyes are organic dyes which carry a net positive charge. They deposit onto cotton. They are of particular utility for used in composition that contain predominantly cationic surfactants. Dyes may be selected from the basic violet and basic blue dyes listed in the Colour Index International.

Preferred examples include triarylmethane basic dyes, methane basic dye, anthraquinone basic dyes, basic blue 16, basic blue 65, basic blue 66, basic blue 67, basic blue 71, basic blue 159, basic violet 19, basic violet 35, basic violet 38, basic violet 48; basic blue 3, basic blue 75, basic blue 95, basic blue 122, basic blue 124, basic blue 141.

### Reactive dyes:

Reactive dyes are dyes which contain an organic group capable of reacting with cellulose and linking the dye to cellulose with a covalent bond. They deposit onto cotton.

Preferably the reactive group is hydrolysed or reactive group of the dyes has been reacted with an organic species for example a polymer, so as to the link the dye to this species. Dyes may be selected from the reactive violet and reactive blue dyes listed in the Colour Index International.

Preferred examples include reactive blue 19, reactive blue 163, reactive blue 182 and reactive blue, reactive blue 96.

### Dye conjugates:

Dye conjugates are formed by binding direct, acid or basic dyes to polymers or particles via physical forces. Dependent on the choice of polymer or particle they deposit on cotton or synthetics. A description is given in WO2006/055787.

Particularly preferred dyes are: direct violet 7, direct violet 9, direct violet 11, direct violet 26, direct violet 31, direct violet 35, direct violet 40, direct violet 41, direct violet 51, direct violet 99, acid blue 98, acid violet 50, acid blue 59, acid violet 17, acid black 1, acid blue 29, solvent violet 13, disperse violet 27 disperse violet 26, disperse violet 28, disperse violet 63, disperse violet 77 and mixtures thereof.

Shading dye can be used in the absence of fluorescent agents, but it is especially preferred to use a shading dye in combination with a fluorescent agent, for example in order to reduce yellowing due to chemical changes in adsorbed fluorescent agents.

### External Structurants

Compositions of the invention may have their rheology further modified by use of one or more external structurants which form a structuring network within the composition. Examples of such materials include hydrogenated castor oil, microfibrous cellulose and citrus pulp fibre. The presence of an external structurant may provide shear thinning rheology and may also enable materials such as encapsulates and visual cues to be suspended stably in the liquid.

### Fragrances

Fragrances are well known in the art and may be incorporated into compositions described herein. Preferably the fragrance is provided as a fragrance oil and more preferably, the fragrance oil is used as a carrier for the additional anti-oxidant when used.

Preferably, the fragrance comprises a phenolic and/or ketonic species.

### Microcapsules

One type of microparticle suitable for use in the invention is a microcapsule. Microencapsulation may be defined as the process of surrounding or enveloping one substance within another substance on a very small scale, yielding capsules ranging from less than one micron to several hundred microns in size. The material that is encapsulated may be called the core, the active ingredient or agent, fill, payload, nucleus, or internal phase. The material encapsulating the core may be referred to as the coating, membrane, shell, or wall material.

Microcapsules typically have at least one generally spherical continuous shell surrounding the core. The shell may contain pores, vacancies or interstitial openings depending on the materials and encapsulation techniques employed. Multiple shells may be made of the same or different encapsulating materials, and may be arranged in strata of varying thicknesses around the core. Alternatively, the microcapsules may be asymmetrically and variably shaped with a quantity of smaller droplets of core material embedded throughout the microcapsule.

The shell may have a barrier function protecting the core material from the environment external to the microcapsule, but it may also act as a means of modulating the release of core materials such as fragrance. Thus, a shell may be water soluble or water swellable and fragrance release may be actuated in response to exposure of the microcapsules to a moist environment. Similarly, if a shell is temperature sensitive, a microcapsule might release fragrance in response to elevated temperatures. Microcapsules may also release fragrance in response to shear forces applied to the surface of the microcapsules.

A preferred type of polymeric microparticle suitable for use in the invention is a polymeric core-shell microcapsule in which at least one generally spherical continuous shell of polymeric material surrounds a core containing the fragrance formulation (f2). The shell will typically comprise at most 20% by weight based on the total weight of the microcapsule. The fragrance formulation (f2) will typically comprise from about 10 to about 60% and preferably from about 20 to about 40% by weight based on the total weight of the microcapsule. The amount of fragrance (f2) may be measured by taking a slurry of the microcapsules, extracting into ethanol and measuring by liquid chromatography.

Polymeric core-shell microcapsules for use in the invention may be prepared using methods known to those skilled in the art such as coacervation, interfacial polymerization, and polycondensation.

The process of coacervation typically involves encapsulation of a generally water-insoluble core material by the precipitation of colloidal material(s) onto the surface of droplets of the material. Coacervation may be simple e.g. using one colloid such as gelatin, or complex where two or possibly more colloids of opposite charge, such as gelatin and gum arabic or gelatin and carboxymethyl cellulose, are used under carefully controlled conditions of pH, temperature and concentration.

Interfacial polymerisation typically proceeds with the formation of a fine dispersion of oil droplets (the oil droplets containing the core material) in an aqueous continuous phase. The dispersed droplets form the core of the future microcapsule and the dimensions of the dispersed droplets directly determine the size of the subsequent microcapsules. Microcapsule shell-forming materials (monomers or oligomers) are contained in both the dispersed phase (oil droplets) and the aqueous continuous phase and they react together at the phase interface to build a polymeric wall around the oil droplets thereby to encapsulate the droplets and form core-shell microcapsules. An example of a core-shell microcapsule produced by this method is a polyurea microcapsule with a shell formed by reaction of diisocyanates or polyisocyanates with diamines or polyamines.

Polycondensation involves forming a dispersion or emulsion of the core material in an aqueous solution of precondensate of polymeric materials under appropriate conditions of agitation to produce capsules of a desired size, and adjusting the reaction conditions to cause condensation of the precondensate by acid catalysis, resulting in the condensate separating from solution and surrounding the dispersed core material to produce a coherent film and the desired microcapsules. An example of a core-shell microcapsule produced by this method is an aminoplast microcapsule with a shell formed from the polycondensation product of melamine (2,4,6-triamino-1,3,5-triazine) or urea with formaldehyde. Suitable cross-linking agents (e.g. toluene diisocyanate, divinyl benzene, butanediol diacrylate) may also be used and secondary wall polymers may also be used as appropriate, e.g. anhydrides and their derivatives, particularly polymers and co-polymers of maleic anhydride.

One example of a preferred polymeric core-shell microcapsule for use in the invention is an aminoplast microcapsule with an aminoplast shell surrounding a core containing the fragrance formulation (f2). More preferably such an aminoplast shell is formed from the polycondensation product of melamine with formaldehyde.

Polymeric microparticles suitable for use in the invention will generally have an average particle size between 100 nanometers and 50 microns. Particles larger than this are entering the visible range. Examples of particles in the sub-micron range include latexes and mini-emulsions with a typical size range of 100 to 600 nanometers. The preferred particle size range is in the micron range. Examples of particles in the micron range include polymeric core-shell microcapsules (such as those further described above) with a typical size range of 1 to 50 microns, preferably 5 to 30 microns. The average particle size can be determined by light scattering using a Malvern Mastersizer with the average particle size being taken as the median particle size D (0.5) value. The particle size distribution can be narrow, broad or multimodal. If necessary, the microcapsules as initially produced may be filtered or screened to produce a product of greater size uniformity.

Polymeric microparticles suitable for use in the invention may be provided with a deposition aid at the outer surface of the microparticle. Deposition aids serve to modify the properties of the exterior of the microparticle, for example to make the microparticle more substantive to a desired substrate. Desired substrates include cellulosics (including cotton) and polyesters (including those employed in the manufacture of polyester fabrics).

The deposition aid may suitably be provided at the outer surface of the microparticle by means of covalent bonding, entanglement or strong adsorption. Examples include polymeric core-shell microcapsules (such as those further described above) in which a deposition aid is attached to the outside of the shell, preferably by means of covalent bonding. While it is preferred that the deposition aid is attached directly to the outside of the shell, it may also be attached via a linking species.

Deposition aids for use in the invention may suitably be selected from polysaccharides having an affinity for cellulose. Such polysaccharides may be naturally occurring or synthetic and may have an intrinsic affinity for cellulose or may have been derivatised or otherwise modified to have an affinity for cellulose. Suitable polysaccharides have a 1-4 linked β glycan (generalised sugar) backbone structure with at least 4, and preferably at least 10 backbone residues which are β1-4 linked, such as a glucan backbone (consisting of β1-4 linked glucose residues), a mannan backbone (consisting of β1-4 linked mannose residues) or a xylan backbone (consisting of β1-4 linked xylose residues). Examples of such β1-4 linked polysaccharides include xyloglucans, glucomannans, mannans, galactomannans, β(1-3),(1-4) glucan and the xylan family incorporating glucurono-, arabino- and glucuronoarabinoxylans. Preferred β1-4 linked polysaccharides for use in the invention may be selected from xyloglucans of plant origin, such as pea xyloglucan and tamarind seed xyloglucan (TXG) (which has a β1-4 linked glucan backbone with side chains of α-D xylopyranose and β-D-galactopyranosyl-(1-2)-α-D-xylopyranose, both 1-6 linked to the backbone); and galactomannans of plant origin such as loc ust bean gum (LBG) (which has a mannan backbone of β1-4 linked mannose residues, with single unit galactose side chains linked α1-6 to the backbone).

Also suitable are polysaccharides which may gain an affinity for cellulose upon hydrolysis, such as cellulose mono-acetate; or modified polysaccharides with an affinity for cellulose such as hydroxypropyl cellulose, hydroxypropyl methylcellulose, hydroxyethyl methylcellulose, hydroxypropyl guar, hydroxyethyl ethylcellulose and methylcellulose.

Deposition aids for use in the invention may also be selected from phthalate containing polymers having an affinity for polyester. Such phthalate containing polymers may have one or more nonionic hydrophilic segments comprising oxyalkylene groups (such as oxyethylene, polyoxyethylene, oxypropylene or polyoxypropylene groups), and one or more hydrophobic segments comprising terephthalate groups. Typically, the oxyalkylene groups will have a degree of polymerization of from 1 to about 400, preferably from 100 to about 350, more preferably from 200 to about 300. A suitable example of a phthalate containing polymer of this type is a copolymer having random blocks of ethylene terephthalate and polyethylene oxide terephthalate.

Mixtures of any of the above described materials may also be suitable.

Deposition aids for use in the invention will generally have a weight average molecular weight (M_{w}) in the range of from about 5 kDa to about 500 kDa, preferably from about 10 kDa to about 500 kDa and more preferably from about 20 kDa to about 300 kDa.

One example of a particularly preferred polymeric core-shell microcapsule for use in the invention is an aminoplast microcapsule with a shell formed by the polycondensation of melamine with formaldehyde; surrounding a core containing the fragrance formulation (f2); in which a deposition aid is attached to the outside of the shell by means of covalent bonding. The preferred deposition aid is selected from β1-4 linked polysaccharides, and in particular the xyloglucans of plant origin, as are further described above.

The present inventors have surprisingly observed that it is possible to reduce the total level of fragrance included in the composition of the invention without sacrificing the overall fragrance experience delivered to the consumer at key stages in the laundry process. A reduction in the total level of fragrance is advantageous for cost and environmental reasons.

Accordingly, the total amount of fragrance formulation (f1) and fragrance formulation (f2) in the concentrated laundry composition of the invention suitably ranges from 0.5 to 1.4%, preferably from 0.5 to 1.2%, more preferably from 0.5 to 1% and most preferably from 0.6 to 0.9% (by weight based on the total weight of the concentrated laundry composition).

The weight ratio of fragrance formulation (f1) to fragrance formulation (f2) in the composition of the invention preferably ranges from 60:40 to 45:55. Particularly good results have been obtained at a weight ratio of fragrance formulation (f1) to fragrance formulation (f2) of around 50:50.

The fragrance (f1) and fragrance (f2) are typically incorporated at different stages of formation of the composition of the invention. Typically, the discrete polymeric microparticles (e.g. microcapsules) entrapping fragrance formulation (f2) are added in the form of a slurry to a warmed base formulation comprising other components of the composition (such as surfactants and solvents). Fragrance (f1) is typically post-dosed later after the base formulation has cooled.

### Other ingredients

The composition may contain further optional ingredients to enhance performance and/or consumer acceptability. Examples of such ingredients include foam boosting agents, polyelectrolytes, anti-shrinking agents, anti-wrinkle agents, anti-oxidants, sunscreens, anticorrosion agents, drape imparting agents, anti-static agents, ironing aids, colorants, pearlisers and/or opacifiers. Each of these ingredients will be present in an amount effective to accomplish its purpose. Generally, these optional ingredients are included individually at an amount of up to 5% based on total weight of the composition.

Many of the ingredients used in embodiments of the invention may be obtained from so called black carbon sources or a more sustainable green source. The following provides a list of alternative sources for several of these ingredients and how they can be made into raw materials described herein.

### SLES and PAS

SLES and other such alkali metal alkyl ether sulphate anionic surfactants are typically obtainable by sulphating alcohol ethoxylates. These alcohol ethoxylates are typically obtainable by ethoxylating linear alcohols. Similarly, primary alkyl sulphate surfactants (PAS) can be obtained from linear alcohols directly by sulphating the linear alcohol. Accordingly, forming the linear alcohol is a central step in obtaining both PAS and alkali-metal alkyl ether sulphate surfactants.

The linear alcohols which are suitable as an intermediate step in the manufacture of alcohol ethoxylates and therefore anionic surfactants such as sodium lauryl ether sulphate ca be obtained from many different sustainable sources. These include:

### Primary sugars

Primary sugars are obtained from cane sugar or sugar beet, etc., and may be fermented to form bioethanol. The bioethanol is then dehydrated to form bio-ethylene which then undergoes olefin methathesis to form alkenes. These alkenes are then processed into linear alcohols either by hydroformylation or oxidation.

An alternative process also using primary sugars to form linear alcohols can be used and where the primary sugar undergoes microbial conversion by algae to form triglycerides. These triglycerides are then hydrolysed to linear fatty acids and which are then reduced to form the linear alcohols.

### Biomass

Biomass, for example forestry products, rice husks and straw to name a few may be processed into syngas by gasification. Through a *Fischer Tropsch* reaction these are processed into alkanes, which in turn are dehydrogenated to form olefins. These olefins may be processed in the same manner as the alkenes described above [primary sugars].

An alternative process turns the same biomass into polysaccharides by steam explosion which may be enzymatically degraded into secondary sugars. These secondary sugars are then fermented to form bioethanol which in turn is dehydrated to form bio-ethylene. This bio-ethylene is then processed into linear alcohols as described above [primary sugars].

### Waste Plastics

Waste plastic is pyrolyzed to form pyrolysed oils. This is then fractioned to form linear alkanes which are dehydrogenated to form alkenes. These alkenes are processed as described above [primary sugars].

Alternatively, the pyrolyzed oils are cracked to form ethylene which is then processed to form the required alkenes by olefin metathesis. These are then processed into linear alcohols as described above [primary sugars].

### Municipal Solid Waste

MSW is turned into syngas by gasification. From syngas it may be processed as described above [primary sugars] or it may be turned into ethanol by enzymatic processes before being dehydrogenated into ethylene. The ethylene may then be turned into linear alcohols by the *Ziegler Process.*

The MSW may also be turned into pyrolysis oil by gasification and then fractioned to form alkanes. These alkanes are then dehydrogenated to form olefins and then linear alcohols.

### Marine Carbon

There are various carbon sources from marine flora such as seaweed and kelp. From such marine flora the triglycerides can be separated from the source and which is then hydrolysed to form the fatty acids which are reduced to linear alcohols in the usual manner.

Alternatively, the raw material can be separated into polysaccharides which are enzymatically degraded to form secondary sugars. These may be fermented to form bio-ethanol and then processed as described above [Primary Sugars].

### Waste Oils

Waste oils such as used cooking oil can be physically separated into the triglycerides which are split to form linear fatty acids and then linear alcohols as described above.

Alternatively, the used cooking oil may be subjected to the Neste Process whereby the oil is catalytically cracked to form bio-ethylene. This is then processed as described above.

### Methane Capture

Methane capture methods capture methane from landfill sites or from fossil fuel production. The methane may be formed into syngas by gasification. The syngas may be processed as described above whereby the syngas is turned into methanol (*Fischer Tropsch* reaction) and then olefins before being turned into linear alcohols by hydroformylation oxidation.

Alternatively, the syngas may be turned into alkanes and then olefins by *Fischer Tropsch* and then dehydrogenation.

### Carbon Capture

Carbon dioxide may be captured by any of a variety of processes which are all well known. The carbon dioxide may be turned into carbon monoxide by a reverse water gas shift reaction and which in turn may be turned into syngas using hydrogen gas in an electrolytic reaction. The syngas is then processed as described above and is either turned into methanol and/or alkanes before being reacted to form olefins.

Alternatively, the captured carbon dioxide is mixed with hydrogen gas before being enzymatically processed to form ethanol. This is a process which has been developed by Lanzatech. From here the ethanol is turned into ethylene and then processed into olefins and then linear alcohols as described above.

The above processes may also be used to obtain the C16/C18 chains of the C16/18 alcohol ethoxylate and/or the C16/C18 ether sulfates.

### Packaging and dosing

The composition may be formulated into any suitable physical form, including powders, granulates, tablets, liquids, etc. Preferably, the composition is provided in a liquid form. More preferably the composition is a liquid detergent composition such as a liquid laundry composition or a liquid dishwash composition, even more preferably the composition is a liquid laundry composition.

The composition of the invention may be supplied in multidose plastics packs with a top or bottom closure. A dosing measure may be supplied with the pack either as a part of the cap or as an integrated system.

Alternatively, the composition of the invention may be packaged as unit doses in polymeric film soluble in the wash water. The unit dose composition of the invention is contained within a pouch formed by a water dissoluble film. Preferably, the pouch has from one to four compartments. More preferably, the pouch has three compartments. It is preferred that the pouch is a unit dose of product and may be from 10 to 50 g in weight to represent a unit dose.

Such water-soluble film compositions, optional ingredients for use therein, and methods of making the same are well known in the art, whether being used for making relatively thin water-soluble films (e.g., as pouch materials) or otherwise.

In one class of embodiments, the water-soluble film includes a water dissoluble material. Preferred such materials include polyvinyl alcohol (PVOH), including homopolymers thereof (e.g., including substantially only vinyl alcohol and vinyl acetate monomer units) and copolymers thereof (e.g., including one or more other monomer units in addition to vinyl alcohol and vinyl acetate units). PVOH is a synthetic resin generally prepared by the alcoholysis, usually termed hydrolysis or saponification, of polyvinyl acetate. Fully hydrolyzed PVOH, wherein virtually all the acetate groups have been converted to alcohol groups, is a strongly hydrogen-bonded, highly crystalline polymer which dissolves only in hot water- greater than about 140 degrees Fahrenheit (60 degrees C). If a sufficient number of acetate groups are allowed to remain after the hydrolysis of polyvinyl acetate, the PVOH polymer then being known as partially hydrolyzed, it is more weakly hydrogen-bonded and less crystalline and is soluble in cold water- less than about 50 degrees Fahrenheit (10 degrees C). An intermediate cold or hot water soluble film can include, for example, intermediate partially- hydrolyzed PVOH (e.g., with degrees of hydrolysis of about 94 percent to about 98 percent), and is readily soluble only in warm water- e.g., rapid dissolution at temperatures of about 40 degrees centigrade and greater. Both fully and partially hydrolyzed PVOH types are commonly referred to as PVOH homopolymers although the partially hydrolyzed type is technically a vinyl alcohol- vinyl acetate copolymer.

The degree of hydrolysis (DH) of the PVOH polymers and PVOH copolymers included in the water-soluble films of the present disclosure can be in a range of about 75 percent to about 99 percent (e.g., about 79 percent to about 92 percent, about 86.5 percent to about 89 percent, or about 88 percent, such as for cold-water soluble compositions; about 90 percent to about 99 percent, about 92 percent to about 99 percent, or about 95 percent to about 99 percent). As the degree of hydrolysis is reduced, a film made from the resin will have reduced mechanical strength but faster solubility at temperatures below about 20 degrees centigrade As the degree of hydrolysis increases, a film made from the polymer will tend to be mechanically stronger and the thermoformability will tend to decrease. The degree of hydrolysis of the PVOH can be chosen such that the water- solubility of the polymer is temperature dependent, and thus the solubility of a film made from the polymer, any compatibilizer polymer, and additional ingredients is also influenced. In one option the film is cold water-soluble. A cold water-soluble film, soluble in water at a temperature of less than 10 degrees centigrade, can include PVOH with a degree of hydrolysis in a range of about 75 percent to about 90 percent, or in a range of about 80 percent to about 90 percent, or in a range of about 85 percent to about 90 percent. In another option the film is hot water-soluble. A hot water-soluble film, soluble in water at a temperature of at least about 60 degrees centigrade, can include PVOH with a degree of hydrolysis of at least about 98 percent.

Other water soluble polymers for use in addition to the PVOH polymers and PVOH copolymers in the blend can include, but are not limited to modified polyvinyl alcohols, polyacrylates, water-soluble acrylate copolymers, polyvinyl pyrrolidone, polyethyleneimine, pullulan, water-soluble natural polymers including, but not limited to, guar gum, gum Acacia, xanthan gum, carrageenan, and starch, water-soluble polymer derivatives including, but not limited to, modified starches, ethoxylated starch, and hydroxypropylated starch, copolymers of the forgoing and combinations of any of the foregoing. Yet other water-soluble polymers can include polyalkylene oxides, polyacrylamides, polyacrylic acids and salts thereof, celluloses, cellulose ethers, cellulose esters, cellulose amides, polyvinyl acetates, polycarboxylic acids and salts thereof, polyaminoacids, polyamides, gelatines, methylcelluloses, carboxymethylcelluloses and salts thereof, dextrins, ethylcelluloses, hydroxyethyl celluloses, hydroxypropyl methylcelluloses, maltodextrins, and polymethacrylates. Such water-soluble polymers, whether PVOH or otherwise are commercially available from a variety of sources. Any of the foregoing water-soluble polymers are generally suitable for use as film-forming polymers. In general, the water-soluble film can include copolymers and/or blends of the foregoing resins.

The water-soluble polymers (e.g., the PVOH resin blend alone or in combination with other water-soluble polymers) can be included in the film in an amount in a range of about 30 weight percent or 50 weight percent to about 90 weight percent or 95 weight percent, for example. The weight ratio of the amount of all water-soluble polymers as compared to the combined amount of all plasticizers, compatibilizing agents, and secondary additives can be in a range of about 0.5 to about 18, about 0.5 to about 15, about 0.5 to about 9, about 0.5 to about 5, about 1 to 3, or about 1 to 2, for example. The specific amounts of plasticizers and other non-polymer component can be selected in a particular embodiment based on an intended application of the water-soluble film to adjust film flexibility and to impart processing benefits in view of desired mechanical film properties.

Water-soluble polymers for use in the film described herein (including, but not limited to PVOH polymers and PVOH copolymers) can be characterized by a viscosity in a range of about 3.0 to about 27.0 mPa s (cP), about 4.0 to about 24.0 cP, about 4.0 to about 23.0 cP, about 4.0 cP to about 15 cP, or about 6.0 to about 10.0 cP, for example. The viscosity of a polymer is determined by measuring a freshly made solution using a Brookfield LV type viscometer with UL adapter as described in British Standard EN ISO 15023-2:2006 Annex E Brookfield Test method. It is international practice to state the viscosity of 4 percent aqueous polyvinyl alcohol solutions at 20 degrees centigrade Polymeric viscosities specified herein in cP should be understood to refer to the viscosity of a 4 percent aqueous water-soluble polymer solution at 20 degrees centigrade, unless specified otherwise.

It is well known in the art that the viscosity of a water-soluble polymer (PVOH or otherwise) is correlated with the weight- average molecular weig ht (W) of the same polymer, and often the viscosity is used as a proxy for Mw. Thus, the weight- average molecular weight of the water-soluble polymers, including the first PVOH copolymer and second PVOH polymer, can be in a range of about 30,000 to about 175,000, or about 30,000 to about 100,000, or about 55,000 to about 80,000, for example.

The water-soluble film can contain other auxiliary agents and processing agents, such as, but not limited to, plasticizers, plasticizer compatibilizers, surfactants, lubricants, release agents, fillers, extenders, cross-linking agents, antiblocking agents, antioxidants, detackifying agents, antifoams, nanoparticles such as layered silicate-type nanoclays (e.g., sodium montmorillonite), bleaching agents (e.g., sodium metabisulfite, sodium bisulfite or others), aversive agents such as bitterants (e.g., denatonium salts such as denatonium benzoate, denatonium saccharide, and denatonium chloride; sucrose octaacetate; quinine; flavonoids such as quercetin and naringen; and quassinoids such as quassin and brucine) and pungents (e.g., capsaicin, piperine, allyl isothiocyanate, and resinferatoxin), and other functional ingredients, in amounts suitable for their intended purposes. Embodiments including plasticizers are preferred. The amount of such agents can be up to about 50 wt., 20 wt percent, 15 wt percent, 10 wt percent, 5 wt percent, 4 wt percent and/or at least 0.01 wt percent, 0.1 wt percent, 1 wt percent, or 5 wt, individually or collectively.

The plasticizer can include, but is not limited to, glycerin, diglycerin, sorbitol, ethylene glycol, diethylene glycol, triethylene glycol, dipropylene glycol, tetraethylene glycol, propylene glycol, polyethylene glycols up to 400 MW, neopentyl glycol, trimethylolpropane, polyether polyols, sorbitol, 2-methyl-I,3-propanediol, ethanolamines, and a mixture thereof. A preferred plasticizer is glycerin, sorbitol, triethyleneglycol, propylene glycol, diproyplene glycol, 2-methyl-I,3-propanediol, trimethylolpropane, or a combination thereof. The total amount of the plasticizer can be in a range of about 10 weight percent to about 40 wt., or about 15 weight percent to about 35 wt., or about 20 weight percent to about 30 wt., for example about 25 wt., based on total film weight. Combinations of glycerin, dipropylene glycol, and sorbitol can be used. Optionally, glycerin can be used in an amount of about 5 wt percent to about 30 wt, or 5 wt percent to about 20 wt, e.g., about 13 wt percent.

Optionally, dipropylene glycol can be used in an amount of about 1 weight percent to about 20 wt., or about 3 weight percent to about 10 wt., for example 6 weight percent. Optionally, sorbitol can be used in an amount of about 1 wt percent to about 20 wt, or about 2 wt percent to about 10 wt, e.g., about 5 wt percent. The specific amounts of plasticizers can be selected in a particular embodiment based on desired film flexibility and processability features of the water-soluble film. At low plasticizer levels, films may become brittle, difficult to process, or prone to breaking. At elevated plasticizer levels, films may be too soft, weak, or difficult to process for a desired use.

In a preferred embodiment the composition comprises a taste aversive such as denatonium benzoate and/or a pungent agent such as capsaicin.

### Examples

### Example 1

### Materials

Protease: Carnival Evity 16L
Amylase: Amplify Prime 100L
Lipase: Lipex 100L
Pectate lyase: Xpect 1000L/T
Cellulase: Celluclean 5000L
Cardanol polyoxyethylene ether sulfate: NSN3003 from Nasurfar Biomaterial Technology Co. Ltd

### Preparation of compositions

Surfactants linear alkylbenzene sulfonate (LAS) and cardanol polyoxyethylene ether sulfate (CES) were dissolved in deionized (DI) water respectively to prepare 15wt% aqueous solution. Pure DI water was used as control. All solutions were freshly prepared and checked to ensure complete dissolution of surfactants.

Compositions were prepared by adding various enzymes into the above-mentioned aqueous solutions. The dosage of enzymes was shown in table 1.

**Table 1**

| **Enzyme** | **Type** | **wt.% in composition** |
|---|---|---|
| Carnival Evity 16L | Protease | 0.5 |
| Amplify Prime 100L | Amylase | 0.2 |
| Lipex 100L | Lipase | 0.3 |
| Xpect 1000L/T | Pectate lyase | 0.2 |
| Celluclean 5000L | Cellulase | 0.3 |

### Method to measure enzyme activity

### Protease activity

The protease activity was measured by a method employing the Suc-AAPF-PNA substrate. Suc-AAPF-PNA is an abbreviation for N-Succinyl-Alanine-Alanine-Proline-Phenylalanine-p-Nitroanilide, and it is a blocked peptide which can be cleaved by endo-proteases. Following the cleavage a free PNA molecule is liberated and it has a yellow color and thus can be measured by visible spectrophotometry at wavelength 405 nm. The increase in absorbance, due to PNA formation, is proportional to the activity of the protease in the sample. The reaction is followed *in situ* and the change in absorbance at 405 nm per time unit is calculated. The protease activity is automatically calculated by referring to a calibration curve of a corresponding reference standard.

### Amylase activity

The amylase activity was measured by a method employing the G7-pNP substrate. G7-pNP which is an abbreviation for 4,6-ethylidene (G7)-p-nitrophenyl (G1)-α, D-maltoheptaoside (ethylidene-G7pNP), a blocked oligosaccharide which can be cleaved by an endo-amylase, such as an α-amylase. The hydrolysed substrate further reacts with α-glucosidase to liberate a free pNP molecule which has a yellow color and thus can be measured by visible spectrophotometry at wavelength 405 nm. The increase in absorbance, due to pNP formation, is proportional to the activity of the amylase in the sample. The reaction is followed *in situ* and the change in absorbance at 405 nm per time unit is calculated. The amylase activity is automatically calculated by referring to a calibration curve of a corresponding reference standard.

### Lipase activity

The lipase activity was measured by a method employing the p-nitrophenyl acyl ester substrate, which can be cleaved by a lipase to liberate a free p-nitrophenol molecule. The p-nitrophenol molecule has a yellow color and thus can be measured by visible spectrophotometry at wavelength 405 nm. The increase in absorbance, due to p-nitrophenol formation, is proportional to the activity of the lipase in the sample. The reaction is followed *in situ* and the change in absorbance at 405 nm per time unit is calculated. The amylase activity is automatically calculated by referring to a calibration curve of a corresponding reference standard.

### Pectate lyase

The pectate lyase activity was measured by a method employing the polygalacturonic acid substrate. Pectate lyase cleaves polygalacturonic acid through a trans elimination mechanism. This means that it leaves a double carbon-carbon bond for each substrate split. This bond absorbs at 235 nm allowing direct detection of pectate lyase activity on soluble polygalacturonic acid by measuring absorbance at that wavelength. The absorbance at 235 nm is measured in a microtiter spectrometer (e.g., Molecular Devices, SpectraMAX 190). The absorbance readings are corrected for background absorbance by subtracting the absorbance of a control sample, run without enzyme added, to all measured values.

### Cellulase

The cellulase activity was measured by a method employing the carboxymethyl cellulose substrate. The substrate carboxymethyl cellulose was hydrolyzed with cellulase at pH 7.5, 50°C for 30 mins. The reaction is stopped by an alkaline reagent containing 4-hydroxybenzhydrazide (PAHBAH) and bismuth which forms complexes with reducing sugar. The complex formation results in color production which can be read at 405 nm by a spectrometer. The increase in absorbance, due to the complex formation, is proportional to the activity of the cellulase in the sample. The reaction is followed *in situ* and the change in absorbance at 405 nm per time unit is calculated. The cellulase activity is automatically calculated by referring to a calibration curve of a corresponding reference standard.

The enzyme activities for the control after preparation (T0) is set at 100%. The compositions were then incubated at 37°C for two weeks and the residual enzyme activities were measured (versus a time T0 for the control, which enzyme activity is set at 100%). The results are shown in table 2.

**Table 2**

| **Enzyme** | **Compositions** | | |
|---|---|---|---|
| | **A (water)** | **B (LAS)** | **1 (CES)** |
| Protease | 15% | 121% | 152% |
| Amylase | 54% | 0% | 57% |
| Lipase | 23% | 1% | 28% |
| Pectate lyase | 48% | 0% | 70% |
| Cellulase | 60% | 1% | 85% |

The results show that composition 1 (according to the invention) provided much better enzyme activities compared to other compositions, indicating improved enzyme stability during storage conditions.

## Claims

1. A composition comprising:
a) an anionically modified alkyl and/or alkenyl phenol polyoxyalkylene ether represented by the formula (I) 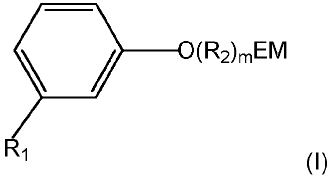 wherein R₁ is a linear or branched, alkyl or alkenyl group having 11 to 21 carbon atoms; each R₂ is an oxyalkylene group having 2 to 4 carbon atoms; m is an integer from 1 to 50; E is a group comprising one or more of sulfate, phosphate, carboxylate, sulfonate, sulfosuccinate, sulfoacetate, sarcosinate and phosphonate; M is a solubilizing cation selected from sodium, potassium, ammonium, mono-, di-, tri-alkanolamine and mixtures thereof; and
b) an enzyme.

2. The composition according to claim 1, wherein R₁ is a linear or branched, alkyl or alkenyl group having 13 to 17 carbon atoms, preferably a linear alkyl or alkenyl group having 13 to 17 carbon atoms.

3. The composition according to claim 1 or claim 2, wherein R₁ is a linear C15 alkyl or alkenyl group, preferably a linear C15 alkyl or alkenyl group comprising 0 to 3 carbon-carbon double bonds.

4. The composition according to any of the preceding claims, wherein each R₂ is an ethylene oxide group.

5. The composition according to any of the preceding claims, wherein m is an integer from 1 to 30, preferably from 2 to 15, more preferably from 3 to 10.

6. The composition according to any of the preceding claims, wherein the anionically modified alkyl and/or alkenyl phenol polyoxyalkylene ether is anionically modified cardanol polyoxyethylene ether.

7. The composition according to any of the preceding claims, wherein the anionically modified alkyl and/or alkenyl phenol polyoxyalkylene ether is cardanol polyoxyethylene ether sulfate, cardanol polyoxyethylene ether phosphate or mixtures thereof, preferably cardanol polyoxyethylene ether sulfate.

8. The composition according to any of the preceding claims, wherein the enzyme comprises protease, lipase, amylase, mannanase, pectate lyase, cellulase, phospholipase, cutinase, peroxidase, oxidase or mixtures thereof.

9. The composition according to any of the preceding claims, wherein the composition comprises the anionically modified alkyl and/or alkenyl phenol polyoxyalkylene ether in an amount from 0.1% to 30% by weight of the composition, preferably from 0.5% to 20%.

10. The composition according to any of the preceding claims, wherein the composition comprises the enzyme in an amount of from 0.00001 to 1%, preferably from 0.0001 to 0.5%.

11. The composition according to any of the preceding claims, wherein the composition further comprises linear alkyl benzene sulfonates.

12. The composition according to any of the preceding claims, wherein the composition is substantially free of alkyl ether sulfates.

13. The composition according to any of the preceding claims, wherein the composition is a detergent composition, preferably a liquid detergent composition, more preferably a liquid laundry detergent composition.

14. The composition according to any of the preceding claims, wherein the composition is in a unit dose format.

15. A method for forming a liquid detergent composition or a wash liquor by dispersing a dose of the composition according to any of the preceding claims in water.

## Patentansprüche

1. Zusammensetzung, umfassend:
a) einen anionisch modifizierten Alkyl- und/oder Alkenylphenolpolyoxyalkylenether der Formel (I) 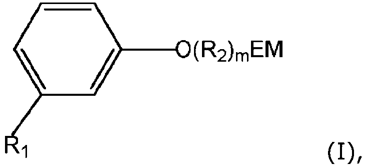 worin
R₁ eine lineare oder verzweigte Alkyl- oder Alkenylgruppe mit 11 bis 21 Kohlenstoffatomen ist,
jedes R₂ eine Oxyalkylengruppe mit 2 bis 4 Kohlenstoffatomen ist;
m eine ganze Zahl von 1 bis 50 ist;
E eine Gruppe ist, die eine oder mehrere der folgenden Gruppen umfasst:
Sulfat, Phosphat, Carboxylat, Sulfonat, Sulfosuccinat, Sulfoacetat,
Sarcosinat und Phosphonat;
M ein solubilisierendes Kation ist, ausgewählt unter Natrium, Kalium, Ammonium, Mono-, Di-, Trialkanolamin und Mischungen davon; und
b) ein Enzym.

2. Zusammensetzung nach Anspruch 1, wobei R₁ eine lineare oder verzweigte Alkyl- oder Alkenylgruppe mit 13 bis 17 Kohlenstoffatomen ist, vorzugsweise eine lineare Alkyl- oder Alkenylgruppe mit 13 bis 17 Kohlenstoffatomen.

3. Zusammensetzung nach Anspruch 1 oder Anspruch 2, wobei R₁ eine lineare C15-Alkyl- oder Alkenylgruppe ist, vorzugsweise eine lineare C15-Alkyl- oder Alkenylgruppe mit 0 bis 3 Kohlenstoff-Kohlenstoff-Doppelbindungen.

4. Zusammensetzung nach einem der vorhergehenden Ansprüche, wobei jedes R₂ eine Ethylenoxidgruppe ist.

5. Zusammensetzung nach einem der vorhergehenden Ansprüche, wobei m eine ganze Zahl von 1 bis 30 ist, vorzugsweise von 2 bis 15, bevorzugter von 3 bis 10.

6. Zusammensetzung nach einem der vorhergehenden Ansprüche, wobei der anionisch modifizierte Alkyl- und/oder Alkenylphenolpolyoxyalkylenether ein anionisch modifizierter Cardanolpolyoxyethylenether ist.

7. Zusammensetzung nach einem der vorhergehenden Ansprüche, wobei der anionisch modifizierte Alkyl- und/oder Alkenylphenolpolyoxyalkylenether Cardanolpolyoxyethylenethersulfat, Cardanolpolyoxyethylenetherphosphat oder Mischungen davon, vorzugsweise Cardanolpolyoxyethylenethersulfat, ist.

8. Zusammensetzung nach einem der vorhergehenden Ansprüche, wobei das Enzym Protease, Lipase, Amylase, Mannanase, Pektatlyase, Cellulase, Phospholipase, Cutinase, Peroxidase, Oxidase oder Mischungen davon umfasst.

9. Zusammensetzung nach einem der vorhergehenden Ansprüche, wobei die Zusammensetzung den anionisch modifizierten Alkyl- und/oder Alkenylphenolpolyoxyalkylenether in einer Menge von 0,1 bis 30 Gew.-% der Zusammensetzung, vorzugsweise von 0,5 bis 20 Gew.-%, umfasst.

10. Zusammensetzung nach einem der vorhergehenden Ansprüche, wobei die Zusammensetzung das Enzym in einer Menge von 0.00001 bis 1%, vorzugsweise von 0,0001 bis 0,5%, umfasst.

11. Zusammensetzung nach einem der vorhergehenden Ansprüche, wobei die Zusammensetzung ferner lineare Alkylbenzolsulfonate umfasst.

12. Zusammensetzung nach einem der vorhergehenden Ansprüche, wobei die Zusammensetzung im Wesentlichen frei von Alkylethersulfaten ist.

13. Zusammensetzung nach einem der vorhergehenden Ansprüche, wobei die Zusammensetzung eine Waschmittelzusammensetzung, vorzugsweise eine flüssige Waschmittelzusammensetzung, bevorzugter eine flüssige Waschmittelzusammensetzung für Wäsche ist.

14. Zusammensetzung nach einem der vorhergehenden Ansprüche, wobei die Zusammensetzung in Einzeldosisform vorliegt.

15. Verfahren zur Herstellung einer flüssigen Waschmittelzusammensetzung oder einer Waschlauge durch Dispergieren einer Dosis der Zusammensetzung nach einem der vorhergehenden Ansprüche in Wasser.

## Revendications

1. Composition comprenant :
a) un éther polyoxyalkylène de phénol d'alkyle et/ou d'alkényle modifié de manière anionique, représenté par la formule (1) 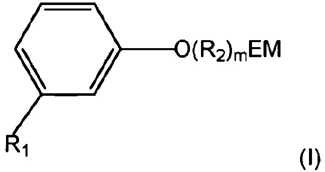 dans laquelle R₁ est un groupe alkyle ou alkényle linéaire ou ramifié ayant de 11 à 21 atomes de carbone ; chaque R₂ est un groupe oxyalkylène ayant de 2 à 4 atomes de carbone ; m est un nombre entier compris entre 1 et 50 ; E est un groupe comprenant un ou plusieurs d'un sulfate, d'un phosphate, d'un carboxylate, d'un sulfonate, d'un sulfosuccinate, d'un sulfoacétate, d'un sarcosinate et d'un phosphonate ; M est un cation solubilisant choisi parmi le sodium, le potassium, l'ammonium, la mono-, di-, tri-alkanolamine et des mélanges de ceux-ci ; et
b) une enzyme.

2. Composition selon la revendication 1, dans laquelle R₁ est un groupe alkyle ou alkényle linéaire ou ramifié ayant de 13 à 17 atomes de carbone, de préférence un groupe alkyle ou alkényle linéaire ayant de 13 à 17 atomes de carbone.

3. Composition selon la revendication 1 ou 2, dans laquelle R₁ est un groupe alkyle ou alkényle linéaire en C15, de préférence un groupe alkyle ou alkényle linéaire en C15 comprenant de 0 à 3 liaisons doubles carbone-carbone.

4. La composition selon l'une quelconque des revendications précédentes, dans laquelle chaque R₂ est un groupe d'oxyde d'éthylène.

5. Composition selon l'une quelconque des revendications précédentes, dans laquelle m est un nombre entier compris entre 1 et 30, de préférence entre 2 et 15, de préférence entre 3 et 10.

6. Composition selon l'une quelconque des revendications précédentes, dans laquelle l'éther polyoxyalkylène de phénol d'alkyle et/ou d'alkényle modifié de manière anionique est un éther polyoxyéthylène de cardanol modifié de manière anionique.

7. Composition selon l'une quelconque des revendications précédentes, dans laquelle l'éther polyoxyalkylène de phénol d'alkyle et/ou d'alkényle modifié de manière anionique est du sulfate d'éther polyoxyéthylène de cardanol, du phosphate d'éther polyoxyéthylène de cardanol ou des mélanges de ceux-ci, de préférence du sulfate d'éther polyoxyéthylène de cardanol.

8. Composition selon l'une quelconque des revendications précédentes, dans laquelle l'enzyme comprend une protéase, une lipase, une amylase, une mannanase, une pectate lyase, une cellulase, une phospholipase, une cutinase, une peroxydase, une oxydase ou des mélanges de celles-ci.

9. Composition selon l'une quelconque des revendications précédentes, dans laquelle la composition comprend de l'éther polyoxyalkylène de phénol d'alkyle et/ou d'alkényle modifié de manière anionique en une quantité comprise entre 0,1 % et 30 % en poids de la composition, de préférence entre 0,5 % et 20 %.

10. Composition selon l'une quelconque des revendications précédentes, dans laquelle la composition comprend l'enzyme en une quantité comprise entre 0,00001 et 1 %, de préférence entre 0,0001 et 0,5 %.

11. Composition selon l'une quelconque des revendications précédentes, dans laquelle la composition comprend en outre des alkylbenzènesulfonates linéaires.

12. Composition selon l'une quelconque des revendications précédentes, dans laquelle la composition est sensiblement exempte de sulfate d'éther alkylé.

13. Composition selon l'une quelconque des revendications précédentes, dans laquelle la composition est une composition détergente, de préférence une composition détergente liquide, de préférence une composition détergente liquide pour le lavage du linge.

14. Composition selon l'une quelconque des revendications précédentes, dans laquelle la composition se présente sous la forme d'une dose unitaire.

15. Procédé de formation d'une composition détergente liquide ou d'une liqueur de lavage en dispersant une dose de la composition selon l'une quelconque des revendications précédentes dans de l'eau.
